# EUROPEAN PATENT APPLICATION

(11) **EP 4 804 202 A1**
(43) Date of publication of application: **09.09.2026**
(21) Application number: 24901069.5
(22) Date of filing: 04.12.2024
(51) Int. Cl.: G16H 40/40, G16H 40/20

(54) **APPARATUS, METHOD, AND COMPUTER PROGRAM FOR MANAGING ON-PREMISE SERVER INSTALLED IN MEDICAL FACILITY**

(30) Priority: 04.12.2023 KR 20230173716
(71) Applicant: Medical AI Co., Ltd., Seoul 06180 (KR)
(72) Inventor: JO, Yongyeon, Seoul 06180 (KR); LEE, Kyuhwan, Seoul 06180 (KR); SHIN, Dongjoo, Seoul 06180 (KR); HAN, Yoon, Seoul 06180 (KR)
(74) Representative: Patentanwaltskanzlei Matschnig & Forsthuber OG
(86) International application number: PCT/KR2024/019758
(87) International publication number: WO 2025/121883

(57) **Abstract**

The present disclosure provides a computing apparatus, a method, and a computer program for managing an on-premises server installed in a medical facility. A method according to an embodiment of the present disclosure comprises receiving status information from a plurality of on-premises servers respectively installed in a plurality of medical facilities, and identifying a target server requiring a software upgrade from among the plurality of on-premises servers, by monitoring the plurality of on-premises servers based on the received status information.

## Description

### TECHNICAL FIELD

The present disclosure relates to an AI (hereinafter "AI") technology in the medical field, and more particularly, to an apparatus, a method, and a computer program for managing an on-premises server installed in a medical facility on which software for analyzing biosignal data of a patient and diagnosing a condition of the patient using an AI technology is installed.

### BACKGROUND OF THE INVENTION

An on-premises server refers to a server directly installed within a user's facility to process and store data, and is suitable for enhancing data security and control authority by minimizing a connection with an external network. In a medical environment, the on-premises server is mainly utilized to process sensitive medical data such as an electrocardiogram, and Magnetic Resonance Imaging (MRI) images, and to support a diagnosis using an AI model. The on-premises server has been widely adopted in medical institutions in that it is possible to process and store data in real time while maintaining data security.

However, a conventional method for managing an on-premises server has several problems. Although a closed network environment is advantageous for enhancing data security, inefficiency occurs in a process of monitoring a server status or updating software due to limited external access. In particular, most AI models executed on an on-premises server are provided from an outside, and a managing entity must directly visit each medical institution to perform an update in order to maintain or improve performance thereof. Accordingly, when a plurality of medical institutions are managed, since each institution must be individually visited and checked, an excessive waste of time and human resources occurs. In addition, a delay in a software update may cause a deterioration in a diagnostic performance of an AI model, and may lead to an inaccurate diagnosis result, which may have a negative effect on a quality of a medical service. As a result, a proper measure for establishing an efficient management system for an on-premises server and AI-based software executed on the on-premises server is required.

### SUMMARY OF THE INVENTION

### TECHNICAL PROBLEM

The present disclosure is contrived in response to the aforementioned background, and an object thereof is to provide an apparatus, a method, and a computer program for managing an on-premises server installed in a medical facility.

However, the problems to be solved by the present disclosure are not limited to the aforementioned problems, and other unmentioned problems will be clearly understood from the following description.

### TECHNICAL SOLUTION

To achieve the aforementioned objects, a method for managing an on-premises server installed in a medical facility, performed by a computing apparatus comprising at least one processor, comprises: receiving status information from a plurality of on-premises servers respectively installed in a plurality of medical facilities; and identifying, from among the plurality of on-premises servers, a target server requiring an upgrade for the software, by monitoring the plurality of on-premises servers based on the received status information.

Alternatively, the method comprises: displaying a flag regarding an update for the software in the setting information of the target server based on the target server being identified,; and transmitting the update data to the target server based on receiving a request for update data for the software from the target server having checked the flag.

Alternatively, the status information comprises hardware resource status information of an on-premises server, comprising at least one of a CPU usage, a memory usage, and a disk usage; and the identifying of a target server comprises identifying, from among the plurality of on-premises servers, a target server requiring an upgrade for the software, by monitoring the performance status of each of the plurality of on-premises servers based on the received resource status information.

Alternatively, the method comprises, when it is identified based on the resource status information received from the target server that the performance of the target server is not improved even after the software of the target server is upgraded, displaying a flag regarding an addition of hardware resources in the setting information of the target server.

Alternatively, the status information comprises service quality status information of the software installed on an on-premises server, comprising at least one of Transactions Per Second (TPS), a response time, and a processing success rate for client requests; and the identifying of a target server comprises identifying, from among the plurality of on-premises servers, a target server requiring an upgrade for the software, by monitoring the quality status of the software of each of the plurality of on-premises servers based on the received resource status information.

Alternatively, the software may be provided by the computing apparatus, and may be configured to calculate, using a neural network model pre-trained with biosignal data acquired from patients, a score for predicting a disease of a patient.

Alternatively, the status information may comprise the calculated score information acquired from each of the on-premises servers; and the identifying of a target server may comprise identifying, from among the plurality of on-premises servers, a target server requiring an upgrade for the software, by identifying a score trend corresponding to each of the on-premises servers based on the score information, and detecting an abnormality in accuracy of the software based on the identified score trend.

Alternatively, the calculated score information may comprise the biological information of the patient corresponding to the score; and the identifying of a target server may comprise identifying, as a target server, an on-premises server in which the patient group characteristics of a medical institution corresponding to each of the on-premises servers are determined to have changed, by determining the patient group characteristics based on the score information.

Alternatively, the method may further comprise displaying a User Interface (UI) indicating a status of each of the on-premises servers via a display based on the received status information.

Alternatively, the plurality of on-premises servers: may communicate with the computing apparatus via a preset port; and based on firewall rules of each of the on-premises servers, may block inbound traffic from the computing apparatus, and may allow only outbound traffic from the on-premises servers.

To achieve the aforementioned objects, a method for acquiring update data for software, the update data being provided by a cloud server managing a plurality of on-premises servers, performed by a computing apparatus comprising at least one processor, may comprise: periodically checking the setting information corresponding to an on-premises server set on the cloud server; and acquiring update data for the software installed on the on-premises server from the cloud server in response to a determination, based on the checked setting information of the on-premises server, that an update for the software is required. A computing apparatus for managing an on-premises server installed in a medical facility, may comprise: a processor comprising at least one core; a memory storing program code executable by the processor; and a communication interface, wherein the processor may be configured to: receive status information from a plurality of on-premises servers respectively installed in a plurality of medical facilities via the communication interface; and identify, from among the plurality of on-premises servers, a target server requiring an upgrade for the software, by monitoring the plurality of on-premises servers based on the received status information.

To achieve the aforementioned objects, a computer program stored on a computer-readable storage medium, when executed by one or more processors, may cause operations for managing an on-premises server installed in a medical facility to be performed. The operations may comprise: receiving status information from a plurality of on-premises servers respectively installed in a plurality of medical facilities; and identifying, from among the plurality of on-premises servers, a target server requiring an upgrade for the software, by monitoring the plurality of on-premises servers based on the received status information.

### ADVANTAGEOUS EFFECTS

According to an embodiment of the present disclosure, a method for managing an on-premises server installed in a medical facility provides an efficient management system for an on-premises server and AI-based software executed on the on-premises server, thereby providing a function of remotely monitoring a server status and updating software even in a closed network environment.

In addition, an inefficient management method that requires a direct visit to a medical institution may be improved, and a waste of time and resources may be reduced.

In addition, by periodically maintaining or improving a performance of an AI model, a performance degradation of software may be prevented, and a reliability of a medical data analysis and a diagnosis result may be improved.

### BRIEF DESCRIPTION OF THE DRAWINGS

FIG. 1 is a diagram illustrating an apparatus for managing an on-premises server installed in a medical facility according to an embodiment of the present disclosure.
FIG. 2 is a block diagram of a computing apparatus according to an embodiment of the present disclosure.
FIG. 3 is a flowchart illustrating a method for managing an on-premises server installed in a medical facility according to an embodiment of the present disclosure.
FIG. 4 is a diagram illustrating a method of displaying a flag indicating whether to update a target server according to an embodiment of the present disclosure.
FIG. 5 is a flowchart illustrating a method of displaying a flag indicating whether to update a target server according to an embodiment of the present disclosure.
FIG. 6 is a diagram illustrating a dashboard for managing a plurality of on-premises servers according to an embodiment of the present disclosure.
FIG. 7 is a block diagram of a computing apparatus according to another embodiment of the present disclosure.

### DETAILED DESCRIPTION OF THE EMBODIMENTS

Hereinafter, the present disclosure describes embodiments in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and is not to be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" is to be understood to mean any of the natural inclusive permutations. For example, if "X uses A or B" is used in the present disclosure, unless otherwise specified or clear from the context, it may be interpreted as any of the cases where X uses A, X uses B, or X uses both A and B.

The term "and/or" as used in the present disclosure is to be understood to refer to and include all possible combinations of one or more of the associated listed concepts.

The terms "comprise" and/or "comprising" as used in the present disclosure are to be understood to mean the presence of a specific feature and/or component. However, the terms "comprise" and/or "comprising" are to be understood as not precluding the presence or addition of one or more other features, other components, and/or combinations thereof.

In the present disclosure, unless otherwise specified or clear from the context to indicate a singular form, generally the singular is to be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "acquire" as used in the present disclosure may be understood as not only receiving data via a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing apparatus or a set thereof, an application program that performs a specific function of software, a processing procedure implemented by software execution, or a set of instructions for program execution. Furthermore, in a broad sense, a "module" or a "unit" may refer to a computing apparatus itself that constitutes a system, or an application executed on the computing apparatus. However, the above-mentioned concepts are only examples, and the concepts of "module" or "unit" may be variously defined within the scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have problem-solving ability by optimizing parameters that connect nodes or neurons through learning. A neural network "model" may include a single neural network or a set of neural networks in which a plurality of neural networks are combined.

The term "data" as used in the present disclosure may include "images", signals, and the like. The term "image" as used in the present disclosure may refer to multi-dimensional data composed of discrete image elements. In other words, an "image" may be understood as a term referring to a digital representation of an object that can be seen by the human eye. For example, an "image" may refer to multi-dimensional data composed of elements corresponding to pixels in a 2D image. An "image" may refer to multi-dimensional data composed of elements corresponding to voxels in a 3D image.

The foregoing description of the terms is for the purpose of aiding the understanding of the present disclosure. Therefore, it is to be noted that if the foregoing terms are not explicitly stated as limiting the content of the present disclosure, they are not used in a sense that limits the technical spirit of the content of the present disclosure.

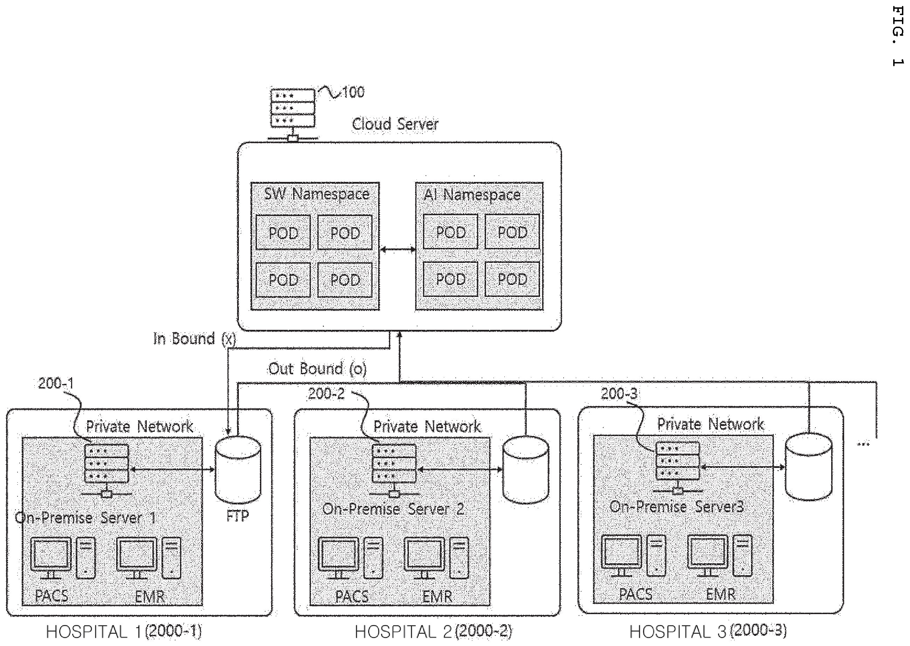

FIG. 1 is a diagram illustrating an apparatus for managing an on-premises server installed in a medical facility 2000 according to an embodiment of the present disclosure.

A computing apparatus 100 according to an embodiment of the present disclosure may be a hardware device or part of a hardware device that performs comprehensive processing and computation of data, or it may be a software-based computing environment connected via a communication interface 130. For example, the computing apparatus 100 may be a server that performs intensive data processing functions and shares resources, or it may be a client that shares resources by interacting with a server. In addition, the computing apparatus 100 may be a cloud system or a cloud server in which a plurality of on-premises servers and clients interact to process data comprehensively. The above description is only one example related to the type of the computing apparatus 100, and therefore, the type of the computing apparatus 100 may be variously configured within the scope understandable by a person of ordinary skill in the art based on the content of the present disclosure. Hereinafter, the computing apparatus 100 is described assuming that the computing apparatus 100 is a cloud server.

Referring to FIG. 1, the computing apparatus 100 may be connected to a plurality of on-premises servers 200-1 to 200-3, hereinafter, 200, respectively installed in a plurality of medical facilities 2000-1 to 2000-3, hereinafter, 2000, via a network to manage the plurality of on-premises servers 200. The on-premises server 200 is a server apparatus installed in the medical facility 2000 to process and store data, and may be used to process data acquired, generated, and managed in the medical facility 2000.

The on-premises server 200 may be installed in an Electronic Medical Record System (EMR) environment (or an electronic medical record system) that electronically records and manages medical information related to a patient's treatment, or a Picture Archiving and Communication System (PACS) system, or may be installed in a medical facility as a separate device (or system) that operates in conjunction with the EMR system and the PACS. The medical facility 2000 may comprise a place that provides medical services to manage and treat a patient's health, such as a hospital, and a public health center.

Software, a program, an application, and the like provided or managed by the computing apparatus 100 may be installed on the on-premises server 200. The computing apparatus 100 may receive information related to the software (or the program, the application, and the like) from the on-premises server 200 to monitor a status of the on-premises server 200. In particular, the computing apparatus 100 may receive status information for identifying the status of the on-premises server 200 from the on-premises server 200 to determine whether an update for software installed on the on-premises server 200 is required. For the on-premises server 200 for which the update is determined to be required, the computing apparatus 100 transmits data required for the update of the corresponding software.

As a result, the computing apparatus 100 according to an embodiment of the present disclosure enables remote monitoring and management of the status of the on-premises server 200 without a manager's direct visit to each medical facility 2000. This may reduce human resources required for managing the on-premises server 200 and provide management efficiency.

Meanwhile, the medical facility 2000 establishes a closed network to prevent a leakage of medical data, enhance security, and ensure a stability of the medical facility 2000 and a medical device from an external intrusion. However, such a closed network makes it practically impossible for the computing apparatus 100 to access the network to monitor or manage the on-premises server 200 of the medical facility 2000. When the network is opened, there is a concern that the aforementioned security and stability may be degraded.

The computing apparatus 100 according to an embodiment of the present disclosure may remotely manage the on-premises server 200 installed in the medical facility 2000 while simultaneously solving the two problems. Specifically, the computing apparatus 100 and the on-premises server 200 communicate via a preset port. The on-premises server 200 allows only outbound traffic to the computing apparatus 100, and blocks inbound traffic from the computing apparatus 100 to the on-premises server 200. This enables communication between the computing apparatus 100 and the on-premises server 200 while maintaining security and stability.

Meanwhile, the computing apparatus 100 may utilize a Pod in a Kubernetes environment to manage each on-premises server 200. A Pod set corresponding to each on-premises server 200 is in charge of communication and management tasks with a specific on-premises server, and may perform management tasks including collecting status information from the on-premises server and updating software. The computing apparatus 100 may separate tasks by creating an independent Pod for each on-premises server, and may secure management efficiency and stability by isolating and dynamically scaling the Pods. For example, the computing apparatus 100 may use a Pod to receive status information from the on-premises server 200 and analyze the same, and when a software update is required for a specific on-premises server, may provide update data suitable for the corresponding server. In this process, the Pod may communicate with a database or a central storage of the computing apparatus 100 to download an update file, or may perform an action based on the status information, and may dynamically create or recover the Pod as needed.

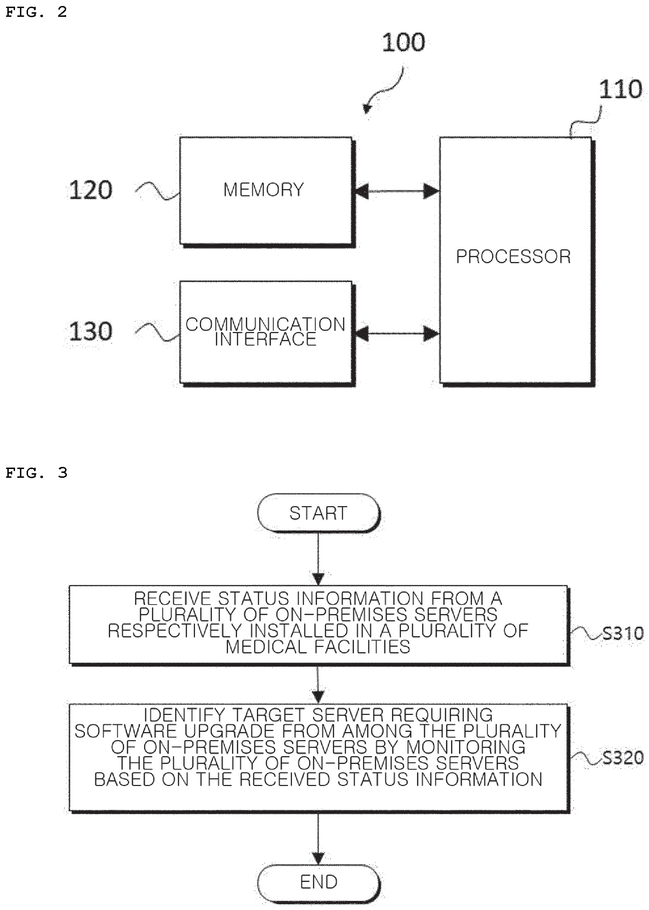

Hereinafter, an embodiment of the present disclosure is described in detail with reference to FIG. 2 to FIG. 6.

FIG. 2 is a block diagram of the computing apparatus 100 according to an embodiment of the present disclosure.

Referring to FIG. 2, the computing apparatus 100 comprises a processor 110 comprising at least one core, a memory 120, and a communication interface 130. However, FIG. 2 is merely an example, and the computing apparatus 100 may further comprise other components for implementing a computing environment. In addition, only some of the disclosed components may be included in the computing apparatus 100.

The processor 110 according to an embodiment of the present disclosure may be understood as a component unit including hardware and/or software for performing computing operations. For example, the processor 110 may execute a computer program and perform data processing for machine learning. The processor 110 may process computational operations such as feature extraction for machine learning and error calculation based on backpropagation. The processor 110 for performing such data processing may include a central processing unit (CPU), a general-purpose graphics processing unit (GPGPU), a tensor processing unit (TPU), an application-specific integrated circuit (ASIC), or a field-programmable gate array (FPGA). The types of the processor 110 described above are only examples, and the type of the processor 110 may be variously configured within the scope understandable by a person of ordinary skill in the art based on the content of the present disclosure.

The processor 110 is electrically connected to other components of the computing apparatus 100 (i.e., the memory 120 and the communication interface 130) to control an overall operation of the computing apparatus 100.

A memory 120 according to an embodiment of the present disclosure may be understood as a constituent unit including hardware and/or software for storing and managing data processed in a computing apparatus 100. That is, the memory 120 may store any form of data generated or determined by the processor 110 and any form of data received by the network unit of the computing apparatus 100. For example, the memory 120 may include at least one storage medium of a flash memory type 120, a hard disk type, a multimedia card micro type, a card type memory 120, a random access memory (RAM), a static random access memory (SRAM), a read-only memory (ROM), an electrically erasable programmable read-only memory (EEPROM), a programmable read-only memory (PROM), a magnetic memory 120, a magnetic disk, or an optical disk. In addition, the memory 120 may include a database system that controls and manages data in a predetermined system. The foregoing types of the memory 120 are only one example, and the type of the memory 120 may be variously configured within a scope understandable by a person of ordinary skill in the art based on the content of the present disclosure.

The memory 120 may structure, organize, and manage data necessary for the processor 110 to perform operations, combinations thereof, and program code executable by the processor 110. For example, the memory 120 may store address information, software information, and setting information of each of the plurality of on-premises servers 200. The setting information may comprise information such as a version of software installed on each on-premises server 200, and a recent update time. In addition, the memory 120 may store a training data set used for training a neural network model used for software, or program code for training the neural network model or operating a pre-trained neural network model, and may store data generated as the program code is executed.

The communication interface 130 according to an embodiment of the present disclosure may be understood as a component unit that transmits and receives data through any form of known wired/wireless communication system. For example, the communication interface 130 may perform data transmission and reception using a wired/wireless communication system such as a local area network (LAN), wideband code division multiple access (WCDMA), long term evolution (LTE), wireless broadband internet (WiBro), 5th generation mobile communication (5G), ultra wideband, ZigBee, radio frequency (RF) communication, wireless LAN, wireless fidelity (Wi-Fi), near field communication (NFC), or Bluetooth. The communication systems described above are only examples, and therefore, wired/wireless communication systems for data transmission and reception of the communication interface 130 may be variously applied in addition to the examples described above.

The communication interface 130 may receive data necessary for the processor 110 to perform a calculation via a wired/wireless communication with any system or any client. In addition, the communication interface 130 may transmit data generated by the calculation of the processor 110 via a wired/wireless communication with any system or any client. For example, the communication interface 130 may receive status information, output data of a neural network model, and the like by communicating with the on-premises server 200 of the medical facility 2000. In addition, the communication interface 130 may transmit data for installing software, update data of software, and the like by communicating with the on-premises server 200.

FIG. 3 is a flowchart illustrating a method for managing an on-premises server installed in a medical facility 2000 according to an embodiment of the present disclosure.

The processor 110 may receive status information from a plurality of on-premises servers 200 respectively installed in a plurality of medical facilities 2000 (S310).

The status information is information indicating a status of the on-premises server 200. The status information may comprise information indicating a status of hardware resources of the on-premises server 200. For example, the status information may comprise at least one of a CPU usage, a memory 120 usage, and a disk usage.

In addition, the status information may comprise information indicating the quality status of a service provided by the on-premises server 200. The service may be a service provided to a client of each medical institution (a medical staff, a patient, and the like) by software provided by the computing apparatus 100. For example, the status information may comprise at least one of Transactions Per Second (TPS), a response time, and a processing success rate for client requests of software.

Meanwhile, the processor 110 may communicate with the plurality of on-premises servers 200 via a preset network port. The preset network port may be a verified port based on a Transmission Control Protocol (TCP). For example, the processor 110 may communicate with the plurality of on-premises servers 200 via a standard port such as an HTTPS (Port 443), an HTTP (Port 80), and an SSH (Port 22) by connecting the computing apparatus 100 and the plurality of on-premises servers 200 via the communication interface 130.

Each of the on-premises servers 200 may block inbound traffic from the computing apparatus 100 and may allow only outbound traffic from the on-premises server 200, based on a firewall rule set in each of the on-premises servers 200. Specifically, the on-premises server 200 may request data from the computing apparatus 100 via outbound traffic, or download an update and setting information of software. The processor 110 may respond to an outbound request of each on-premises server 200 to deliver necessary data, or may support to maintain a latest status of software through status information and an update flag.

Specifically, the on-premises server 200 may request data from the computing apparatus 100 via outbound traffic, or may periodically transmit status information or receive data necessary for a software update. The status information may comprise monitoring indicators such as a hardware status (a CPU usage, a memory 120 usage, a disk usage) and a network performance (TPS, a response time, and the like) of the on-premises server 200 as described above, and the computing apparatus 100 may analyze a performance status of the on-premises server 200 based on such status information to perform a management task such as a visualization of a dashboard to improve a server operation efficiency or a problem detection. Such a traffic control method considers a sensitivity of medical data to fundamentally block an unnecessary access to the on-premises server 200 from an outside, and enables a safe performance of essential management tasks such as a software update and a status monitoring.

Then, the processor 110 may monitor the plurality of on-premises servers 200 based on the received status information, and may identify a target server that requires an upgrade for the software from among the plurality of on-premises servers 200 (S320).

Specifically, the processor 110 may identify a status of each of the on-premises servers 200 based on the status information received from each of the on-premises servers 200. In particular, the processor 110 may monitor hardware resource status of an on-premises server included in the status information to determine whether there is an abnormality in the on-premises server 200. Alternatively, the processor 110 may monitor a quality status of a service included in the status information to determine whether there is an abnormality in the on-premises server 200. That is, whether there is an abnormality in the on-premises server 200 may be determined based on hardware resource status or a service quality status of the on-premises server 200.

In particular, the processor 110 may determine that there is a possibility of a performance degradation or a failure of the on-premises server 200 due to a lack of hardware resources of the on-premises server 200, or may determine that a service quality is degraded, and thus may determine that an abnormality has occurred in the on-premises server 200. In particular, such an abnormality in the on-premises server 200 may be caused by an abnormal operation of software installed on the on-premises server 200. Accordingly, the processor 110 may determine that a software upgrade is required for the on-premises server 200 in which an abnormal status is detected from among the plurality of on-premises servers 200. Hereinafter, the on-premises server 200 in which an abnormal status is detected is referred to as a target server.

According to an embodiment of the present disclosure, the processor 110 may monitor a performance status of each of the plurality of on-premises servers 200 based on received hardware resource status information to identify a target server that requires a software upgrade from among the plurality of on-premises servers 200. Specifically, the processor 110 may determine whether there is an abnormality in the on-premises server 200 by comparing a performance indicator included in the resource status information with a reference value set for each performance indicator. For example, the processor 110 may determine that a status of an on-premises server is an abnormal status when it is identified that a CPU usage of the on-premises server 200 is a preset first value or more, a memory 120 usage is a preset second value or more, or a disk space usage is insufficient as a preset third value or more.

In addition, according to an embodiment of the present disclosure, the processor 110 may monitor a quality status of software of each of the plurality of on-premises servers 200 based on received resource status information to identify a target server that requires a software upgrade from among the plurality of on-premises servers 200.

Specifically, the processor 110 may determine whether there is an abnormality in the on-premises server 200 by comparing a quality indicator included in the service quality status information with a reference value set for each quality indicator. For example, the processor 110 may determine that a status of an on-premises server is an abnormal status when TPS (Transactions Per Second) for software installed on the on-premises server 200 is less than a preset fourth value, a response time of the on-premises server 200 is a preset time or more, or a processing success rate for client requests using the software is less than a preset fifth value. Thereafter, the processor 110 may determine the corresponding on-premises server 200 as a target server.

Meanwhile, the processor 110 may comprehensively determine hardware resources shortage and a software quality degradation to identify an abnormal status of the on-premises server 200.

According to an embodiment of the present disclosure, the status information may comprise score information acquired using software from each of the on-premises servers 200. The processor 110 may identify a score trend corresponding to each of the on-premises servers 200 based on the score information, and may detect an abnormality in accuracy of the software based on the identified score trend to identify a target server that requires a software upgrade from among the plurality of on-premises servers 200.

In relation thereto, according to an embodiment of the present disclosure, the software may be provided by the computing apparatus 100 and installed on each of the on-premises servers 200, and may perform a function of calculating a score for predicting a disease of a patient, using a neural network model pre-trained with biosignal data acquired from patients. The neural network model may be a model trained to calculate a score corresponding to a disease possibility of a patient as an input of biosignal data corresponding to a biosignal measured from the patient. The on-premises server 200 may input biosignal data corresponding to a biosignal of a patient acquired using a biosignal measurement device of a medical institution to the neural network model, and may acquire a score corresponding to a disease possibility of the patient calculated by the neural network model to predict whether the patient has a disease or a possibility of the disease based on the acquired score corresponding to the disease possibility.

The biosignal data may include electrocardiogram (ECG) data corresponding to an electrocardiogram signal acquired from a patient. As an example, the biosignal data may be electrocardiogram data corresponding to an electrocardiogram signal acquired through a 12-lead. However, the present disclosure is not limited thereto, and the biosignal data may include data corresponding to an electroencephalogram (EEG) signal, a body temperature, a blood pressure, a pulse, and the like acquired from a patient. However, for convenience of description of the present disclosure, the biosignal data is assumed to be electrocardiogram data and described below.

Then, a heart disease may be a Left Ventricular Systolic Dysfunction (LVSD). However, the present disclosure is not limited thereto, and a heart disease may include an Atrial Fibrillation, a Ventricular Tachycardia, a Ventricular Fibrillation, a Heart Attack, and the like. In addition, the present disclosure is not limited to a heart disease, and the neural network model may be trained to determine a disease possibility of other body organs other than a heart, such as an Alzheimer's disease, and an Encephalitis, according to a type of biosignal data.

The processor 110 may acquire an input data set comprising a plurality of electrocardiogram data corresponding to electrocardiogram signals acquired from a plurality of patients. In addition, the processor 110 may acquire a label data set comprising a plurality of label data in which a label indicating whether a patient has a left ventricular systolic dysfunction (or a score indicating a degree of the left ventricular systolic dysfunction) is assigned to each of the plurality of electrocardiogram data. Thereafter, the processor 110 may train a neural network model with a training data set comprising the input data set and the label data set. In particular, the processor 110 may train the neural network model with electrocardiogram data and label data corresponding to the same patient included in the input data set and the label data set as a pair.

For example, the processor 110 may acquire a score indicating a degree of a left ventricular systolic dysfunction by inputting electrocardiogram data included in the training data set to the neural network model. The processor 110 may calculate an error using a loss function in which the score acquired through the neural network model and a label of label data paired with the input electrocardiogram data included in the label data set are input variables. The processor 110 may adjust parameters of a neural network included in the neural network model based on the calculated error. Then, the processor 110 may repeatedly perform a process of adjusting the parameters of the neural network until the error satisfies a minimum reference. The training of the neural network model may be performed based on a supervised learning as in the above-described example, but may be performed based on a self-supervised learning according to a structure of the neural network model. Through such a training process, the neural network model may extract feature information related to a waveform of an electrocardiogram signal, such as a P wave, a QRS complex, a T wave, a PR interval, and an RR interval, from electrocardiogram data, and may output a score corresponding to a possibility of a left ventricular systolic dysfunction based on the extracted feature information. As an example, the neural network model may be implemented as a Multi-Layer Perceptron (MLP), a Convolutional Neural Network (CNN), a Recurrent Neural Network (RNN), a Generative Adversarial Network, and the like. Meanwhile, when the training of the neural network model is completed, the processor 110 may distribute software using the neural network model to a plurality of on-premises servers 200 connected to the computing apparatus 100.

The processor 110 may acquire score information calculated by the software as status information from each of the on-premises servers 200. The processor 110 may monitor a score trend corresponding to each of the on-premises servers 200. The processor 110 may monitor a change amount of a score, a calculation amount of a score compared to a preset time, and the like, and may detect an abnormality in accuracy of the software based on the identified score trend.

In particular, the processor 110 may identify a number of patients, an age of patients, a sex of patients, and the like who visit a medical institution in which each of the on-premises servers 200 is installed, based on the score. The processor 110 may consider characteristics of a patient identified for each medical institution, and when a trend of a score changes rapidly, may determine that there is an abnormality in accuracy of the software. In particular, when such a change in the trend of the score is repeated, the processor 110 may determine that there is an abnormality in the accuracy of the software, and may determine the corresponding on-premises server 200 as a target server. In addition, when the processor 110 repeatedly acquires a score as an abnormal value (for example, a score is repeatedly acquired as 0 or 100), the processor 110 may detect an abnormality in accuracy of the software based on the identified score trend. Meanwhile, when the processor 110 detects an abnormality in the software, the processor 110 may identify a cause of the abnormality, and may update the software to solve the abnormality, and may generate data for updating the software.

According to an embodiment of the present disclosure, the calculated score information may comprise biological information of a patient corresponding to the score, and the processor 110 may identify characteristics of a patient group of a medical institution corresponding to each of the on-premises servers 200 based on the score information, and may identify the on-premises server, in which the characteristics of the patient group are determined to have changed, as a target server. In particular, when characteristics of a patient group change, accuracy of a pre-trained neural network model used by software may be degraded, and thus the processor 110 may determine that a software update is required. The processor 110 may train the neural network model using a training data set in which characteristics of a changed patient group are reflected to update the software.

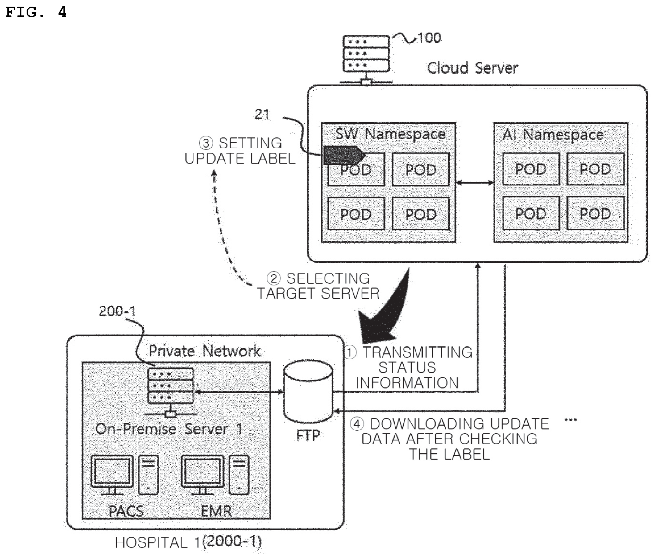

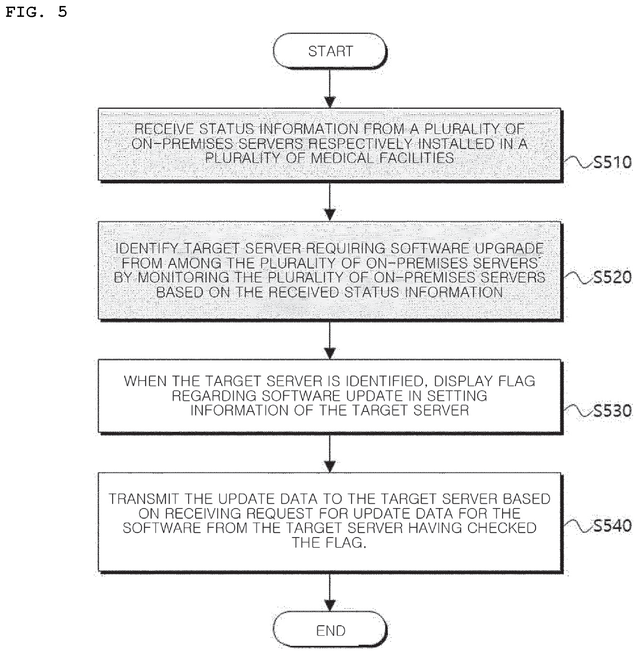

FIG. 4 is a diagram illustrating a method of displaying a flag indicating whether to update a target server according to an embodiment of the present disclosure. FIG. 5 is a flowchart illustrating a method of displaying a flag indicating whether to update a target server according to an embodiment of the present disclosure. S510 and S520 illustrated in FIG. 5 may correspond to S310 and S320 illustrated in FIG. 3, and thus a detailed description thereof is omitted.

According to an embodiment of the present disclosure, when a target server is identified, the processor 110 may display a flag regarding an update for software in the setting information of the target server, and when the processor 110 receives a request for update data for the software from the target server having checked the flag, may transmit the update data to the target server.

Specifically, the processor 110 may create or manage a directory structure corresponding to each of the on-premises servers 200, and the setting information of the on-premises server 200 may be stored in the corresponding directory. The setting information may comprise version information of the software of the on-premises server 200, recent update time information, software update URL information, and the like. Referring to FIG. 4, when a target server is identified, the processor 110 may create a flag 21 in a directory corresponding to the target server to display whether a software update is required. The flag 21 may include a latest version information of software or an item that needs to be updated, and the on-premises server 200 may periodically check the corresponding directory to determine whether an update is required. For example, when the flag 21 is created in the directory corresponding to the target server, the target server starts an update task by this, and requests update data. Thereafter, after receiving a request from the target server, the processor 110 may transmit the software update data to maintain the software of the target server in a latest state. Alternatively, the target server may access a database or a field in which software update data provided by the processor 110 is stored via a software update URL to acquire the software update data. Meanwhile, a determination period of whether an update of the on-premises server 200 is required may be changed according to a time of the last update.

Meanwhile, the processor 110 may manage the flag 21 using a database. For example, when managing the flag 21 based on a database, the processor 110 may distinguish each of the on-premises servers 200 by a unique identifier (Server ID) and store an update flag 21 in a database table. The flag 21 information may comprise data such as a software version, whether an update is required, and a priority, and the on-premises server 200 may access the database to periodically check a set value of the flag 21.

In addition, the processor 110 may display and manage the flag 21 via an API. The processor 110 may provide an API endpoint, and each of the on-premises servers 200 may call the corresponding API to check a value of the update flag 21. For example, the following JSON data may be returned via an API request to check the flag 21.

Meanwhile, according to an embodiment of the present disclosure, the processor 110 may provide update data of software according to the last update time of software of each of the on-premises servers 200 or whether software is updated in the computing apparatus 100. Each of the on-premises servers 200 may periodically access the computing apparatus 100 (or a database of the computing apparatus 100) to monitor whether update data of software is uploaded or whether setting information of each of the on-premises servers 200 is changed. In particular, the setting information may comprise a version of the software installed on the on-premises server 200 and latest version information of the software. When it is determined that software installed on the corresponding server is not the latest version or that software update data is uploaded to the computing apparatus 100, the on-premises server 200 may acquire update data for the latest version of the software. This may be performed for a target server, or may be performed for all of a plurality of on-premises servers without identifying a target server. The update data for the software may be generated in response to an abnormal status of software detected from a specific on-premises server. Meanwhile, a method of the on-premises server 200 acquiring update data or accessing the computing apparatus 100 for this may be equally applied to the description of the above-described embodiment.

Meanwhile, according to an embodiment of the present disclosure, when it is identified based on the resource status information received from a target server that a performance of the target server is not improved even after the software of the target server is upgraded, the processor 110 may display a flag 21 related to an addition of hardware resources in the setting information of the target server. The processor 110 may display another flag 21 indicating that an addition of hardware resources is required in the setting information of the target server. For example, when a CPU usage continuously exceeds a preset first value even after a software upgrade, the processor 110 may determine the corresponding status as a limit of hardware resources and set a hardware expansion flag 21.

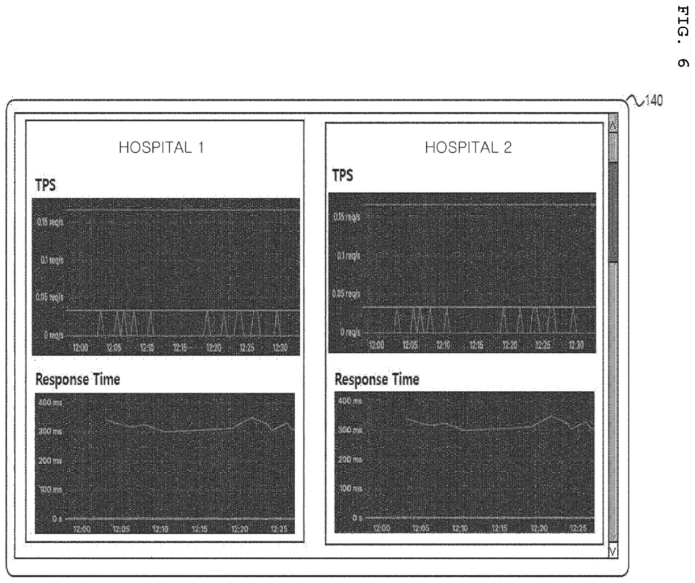

FIG. 6 is a diagram illustrating a dashboard for managing a plurality of on-premises servers 200 according to an embodiment of the present disclosure.

In addition, according to an embodiment of the present disclosure, the processor 110 may display a User Interface (UI) indicating a status of each of the on-premises servers 200 via a display based on status information received from each of the on-premises servers 200. The UI may be designed to allow a real-time monitoring for hardware resource status (a CPU usage, a memory 120 usage, a disk usage), service quality status (TPS, a response time, and the like), update status, and the like of a server. In particular, referring to FIG. 6, the processor may simultaneously display a status of each of the on-premises servers 200 via the display 140 of the computing apparatus 100. The UI comprises visual elements such as a graph, a chart, and a warning message to enable a user to identify a server status at a glance, and may provide a highlight display or a warning notification for a server in which an abnormal status is detected. For example, when a CPU usage of a specific server becomes excessively high, the UI may display the corresponding server in a warning color, and may deliver necessity of a follow-up action such as a hardware resource expansion or a software optimization to a user. Such a UI may contribute to improving a management efficiency by intuitively providing a server status.

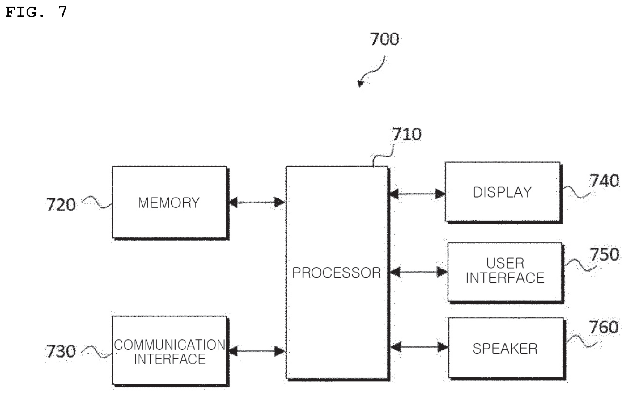

FIG. 7 is a block diagram of a computing apparatus according to another embodiment of the present disclosure.

Referring to FIG. 7, a computing apparatus 700 according to another embodiment of the present disclosure comprises a processor 710, a memory 720, a communication interface 730, a display 740, a user interface 750, and a speaker 760. From among the components illustrated in FIG. 7, the processor 710 and the memory 720 correspond to the processor 110 and the memory 120 of the computing apparatus 100 illustrated in FIG. 2, and thus a detailed description thereof is omitted.

The display 740 may display various images. The images comprise both a still image and a moving image. The display 740 may output guide information about an activity generated based on a user status. The display 740 may be implemented in various forms such as a Liquid Crystal Display Panel (LCD), an Organic Light Emitting Diode (OLED), a Liquid Crystal on Silicon (LCoS), and a Digital Light Processing (DLP). In addition, the display 740 may comprise a driving circuit, a backlight unit, and the like implemented in a form such as an a-si TFT, an LTPS (low temperature poly silicon) TFT, and an OTFT (organic TFT).

Meanwhile, the display 740 may be implemented as a touch screen by being combined with a touch panel, and the display 740 may perform not only an output interface function of outputting an image via the touch screen but also an input interface function of receiving a user's touch input. The display 740 may display status information of a plurality of on-premises servers 200.

The user interface 750 is a component used for an interaction of the computing apparatus 100 with a user, and may comprise at least one of a touch sensor, a motion sensor, a button, a jog dial, and a switch, but is not limited thereto. The processor 710 may receive an on-premises information ID, address information, and the like via the user interface 750.

The speaker 760 is a component that outputs various audio data subjected to various processing tasks such as a decoding, an amplification, and a noise filtering by an audio processing unit (not shown). The speaker 760 may output various notification sounds or voice messages. According to an embodiment of the present disclosure, the processor 710 may convert an electrical signal received from an external device into a user voice and output the same via the speaker 760. As an example, the speaker 760 may output a voice message such as whether a target server is detected, and an identification number of a target server.

Meanwhile, a non-transitory computer readable medium storing a program sequentially performing a method of generating a reading report comprising an analysis result based on AI according to the present disclosure may be provided.

A non-transitory readable medium is a medium that stores data semi-permanently, not for a short period of time such as a register, a cache, and a memory, and is readable by an apparatus. Specifically, the various applications or programs described above may be provided by being stored in a non-transitory readable medium such as a CD, a DVD, a hard disk, a Blu-ray disk, a USB, a memory card, and a ROM.

The various embodiments of the present disclosure described above may be combined with additional embodiments, and may be modified within the scope understandable by a person of ordinary skill in the art in light of the foregoing detailed description. The embodiments of the present disclosure are intended to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts are to be interpreted as being included in the scope of the present disclosure.

## Claims

1. A method for managing an on-premises server installed in a medical facility, performed by a computing apparatus comprising at least one processor, the method comprising:
receiving status information from a plurality of on-premises servers respectively installed in a plurality of medical facilities; and
identifying, from among the plurality of on-premises servers, a target server requiring an upgrade for the software, by monitoring the plurality of on-premises servers based on the received status information.

2. The method of claim 1, comprising:
displaying a flag regarding an update for the software in the setting information of the target server based on the target server being identified; and
transmitting the update data to the target server based on receiving a request for update data for the software from the target server having checked the flag,.

3. The method of claim 1, wherein:
the status information comprises hardware resource status information of an on-premises server, comprising at least one of a CPU usage, a memory usage, and a disk usage; and
the identifying of a target server comprises identifying, from among the plurality of on-premises servers, a target server requiring an upgrade for the software, by monitoring the performance status of each of the plurality of on-premises servers based on the received resource status information.

4. The method of claim 3, comprising:
when it is identified based on the resource status information received from the target server that the performance of the target server is not improved even after the software of the target server is upgraded, displaying a flag regarding an addition of hardware resources in the setting information of the target server.

5. The method of claim 1, wherein:
the status information comprises service quality status information of the software installed on an on-premises server, comprising at least one of Transactions Per Second (TPS), a response time, and a processing success rate for client requests; and
the identifying of a target server comprises identifying, from among the plurality of on-premises servers, a target server requiring an upgrade for the software, by monitoring the quality status of the software of each of the plurality of on-premises servers based on the received resource status information.

6. The method of claim 5, wherein
the software is provided by the computing apparatus, and is configured to calculate, using a neural network model pre-trained with biosignal data acquired from patients, a score for predicting a disease of a patient.

7. The method of claim 6, wherein:
the status information comprises the calculated score information acquired from each of the on-premises servers; and
the identifying of a target server comprises identifying, from among the plurality of on-premises servers, a target server requiring an upgrade for the software, by identifying a score trend or a score abnormality corresponding to each of the on-premises servers based on the score information, and detecting an abnormality in accuracy of the software based on the identified score trend or score abnormality.

8. The method of claim 7, wherein:
the calculated score information comprises the biological information of the patient corresponding to the score; and
the identifying of a target server comprises
identifying, as a target server, an on-premises server in which the patient group characteristics of a medical institution corresponding to each of the on-premises servers are determined to have changed, by determining the patient group characteristics based on the score information.

9. The method of claim 1, comprising
displaying a User Interface (UI) indicating a status of each of the on-premises servers via a display based on the received status information.

10. The method of claim 1, wherein
the plurality of on-premises servers:
communicate with the computing apparatus via a preset port; and
based on firewall rules of each of the on-premises servers, block inbound traffic from the computing apparatus, and allow only outbound traffic from the on-premises servers.

11. A method for acquiring update data for software, the update data being provided by a cloud server managing a plurality of on-premises servers, performed by a computing apparatus comprising at least one processor, the method comprising:
periodically checking the setting information corresponding to an on-premises server set on the cloud server; and
acquiring update data for the software installed on the on-premises server from the cloud server in response to a determination, based on the checked setting information of the on-premises server, that an update for the software is required.

12. A computing apparatus for managing an on-premises server installed in a medical facility, the computing apparatus comprising:
a processor comprising at least one core;
a memory storing program code executable by the processor; and
a communication interface,
wherein the processor is configured to:
receive status information from a plurality of on-premises servers respectively installed in a plurality of medical facilities via the communication interface; and
identify, from among the plurality of on-premises servers, a target server requiring an upgrade for the software, by monitoring the plurality of on-premises servers based on the received status information.

13. A computer program stored on a computer-readable storage medium, the computer program, when executed by one or more processors, causing operations for managing an on-premises server installed in a medical facility to be performed,
the operations comprising:
receiving status information from a plurality of on-premises servers respectively installed in a plurality of medical facilities; and
identifying, from among the plurality of on-premises servers, a target server requiring an upgrade for the software, by monitoring the plurality of on-premises servers based on the received status information.
